# EUROPÄISCHE PATENTSCHRIFT

(11) **EP 1 219 255 B1**
(45) Veröffentlichungstag und Bekanntmachung des Hinweises auf die Patenterteilung: **15.10.2003**
(21) Anmeldenummer: 01106711.3
(22) Anmeldetag: 16.03.2001
(51) Int. Cl.: A61B 17/70

(54) **Schraube zum Verbinden mit einem Stab**
Screw for connection to a rod
Vis pour connexion avec une tige

(30) Priorität: 27.12.2000 DE 10065396
(43) Veröffentlichungstag der Anmeldung: 03.07.2002
(73) Patentinhaber: BIEDERMANN MOTECH GmbH, 78054 Villingen-Schwenningen (DE)
(72) Erfinder: Jeszensky, Dezsö, Dr., 9000 St. Gallen (CH); Biedermann, Lutz, 78054 VS-Schwenningen (DE)
(74) Vertreter: Prüfer, Lutz H., Dipl.-Phys.

(56) Entgegenhaltungen:
- WO-A-00/27297
- DE-A- 19 912 364
- DE-U- 29 810 798
- DE-U- 29 903 342

## Beschreibung

Die Erfindung betrifft ein in der Wirbelsäulen- bzw. Unfallchirurgie zu verwendendes Element zum Verbinden mit einem Stab.

Aus der EP 0 614 649 ist eine Knochenschraube mit einem Schaft und einem damit verbundenen Halteelement zum Verbinden mit einem Stab bekannt. Das Halteelement weist eine Fassung mit einer einen U-förmigen Querschnitt aufweisenden Ausnehmung zur Aufnahme eines Stabes mit zwei an einem Ende freien Schenkeln und einem Innengewinde an den freien Schenkeln auf. Ferner ist eine mit dem Innengewinde zusammenwirkende Schraube, die über ein Druckelement auf einen Kopf des Schaftes zum Blockieren desselben einwirkt, bekannt. Das Innengewinde zeigt im wesentlichen ein Rundgewinde und eine damit zusammenwirkende Innenschraube. Das Gewinde ist als ein oben beschriebenes Rundgewinde ausgebildet. Die Innenschraube wird solange festgezogen, bis sie auf den Stab drückt. Dadurch könnte sich ein Aufbiegen der äußeren freien Schenkel ergeben, was allerdings durch das Aufsetzen einer Außenmutter verhindert wird.

Aus der US 5 005 562 ist es bekannt, bei einer Pedikelschraube nach dem Oberbegriff des Patentanspruchs 1 das Innengewinde und das Gewinde der damit zusammenwirkenden Schraube als Sägezahngewinde auszubilden, um so ein Aufspreizen der beiden Flanken zu vermeiden durch vollständiges Eliminieren der radialen Komponente der Kraft beim Einschrauben.

Ein solches Sägen- oder auch Sägezahngewinde ist aus Meyers Enzyklopädisches Lexikon, Mannheim, 1974, Seite 302 bekannt. Aus den beiden Implantate betreffenden Druckschriften US 5 605 458 und 5 607 304 ist es bekannt, auch bei solchen Implantaten, die im Körper eingebracht werden, insbesondere Hüftimplantaten, das Sägengewinde so auszubilden, daß die die Last tragende Flanke nicht nur unter 90° zur Gewindeachse sondern sogar unter einem negativen Winkel ausgebildet ist. Dadurch soll erreicht werden, daß die mit dem Gewinde erzielte Verbindung einer radialen Relativbewegung der verbundenen Teile widersteht und somit eine verbesserte Fixierung des Implantats gewährleistet. Aus der EP-A-1 128 773 ist eine Pedikelschraube bekannt, bei der das Innengewinde eines eine Stange aufnehmenden Kopfes ein Negativgewinde aufweist und die damit zusammenwirkende Schraube entsprechend ausgebildet ist.

Aus der DE 199 12 364 A1 ist ein Element nach dem Oberbegriff des Patentanspruches 1 bekannt. Aus der DE 299 03 342 U1 ist ein Fixierelement für ein Halteelement eines Wirbelsäulenimplantats bekannt mit einem gabelförmigen Abschnitt in den eine Schraue einschraubbar ist. Das Innengewinde des gabelförmigen Abschnitts und das Außengewinde der Schraube weisen ein Kompressionsgewinde auf, bei dem die tragenden Flanken der Innengewindeabschnitte auf die Öffnung des gabelförmigen Abschnitts hinweisen. Aufgabe der Erfindung ist es, ein Element der eingangs beschriebenen Art zu schaffen, welches geeignet ist, den oben beschriebenen Nachteil zu vermeiden. Insbesondere soll das Aufbiegen der freien Enden einer solchen Fassung und damit ein Lockern der Verbindung zwischen Schraube und Stab vermieden wird.

Diese Aufgabe wird durch das in Patentanspruch 1 gekennzeichnete Element gelöst.

Diese Ausbildung hat den Vorteil, daß die Krafteinwirkung zwischen den freien Schenkeln und der inneren Schraube so geschieht, daß die freien Enden des Halteelementes nicht nach außen drückt, sondern vielmehr nach innen gezogen werden, so daß eine intensive Verbindung zwischen den zusammenwirkenden Elementen geschieht.

Weitere Merkmale und Zweckmäßigkeiten der Erfindung ergeben sich aus der Beschreibung von Ausführungsbeispielen anhand der Figuren. Von den Figuren zeigen:
- Fig. 1: eine Seitenansicht einer monoaxialen Pedikelschraube in geschnittener Explosionsdarstellung;
- Fig. 2: den Kopf der in Fig. 1 gezeigten Pedikelschraube mit eingesetztem Stab und eingesetzter Mutter;
- Fig. 3: das in Fig. 2 angedeutete Detail III in vergrößertem Maßstab;
- Fig. 4: einen Schnitt entsprechend der in Fig. 1 gewählten Darstellung für eine Polyaxialschraube;
- Fig. 5: ein schematisch dargestelltes Halteelement mit eingelegtem Stab und lose eingeschraubter Innenschraube;
- Fig. 6: das Detail VI in Fig. 5 in vergrößertem Maßstab;
- Fig. 7: die der in Fig. 5 gezeigten Darstellung entsprechende Darstellung nach dem Festziehen der Innenschraube; und
- Fig. 8: das in Fig. 7 gezeigte Detail VIII in vergrößertem Maßstab.

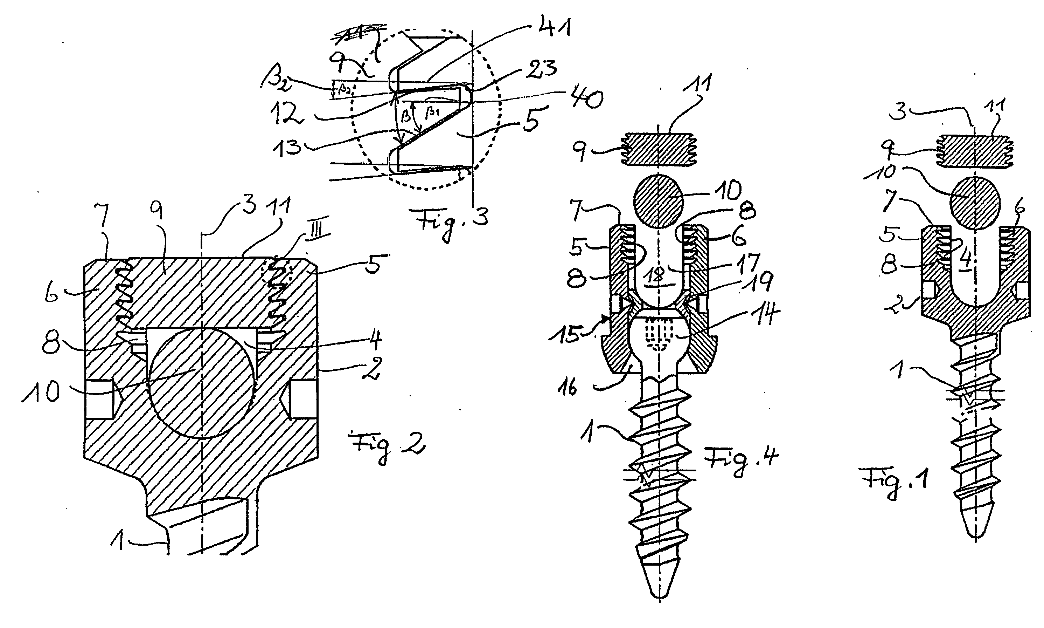

Die Pedikelschraube gemäß Fig. 1, die natürlich auch als Knochenschraube allgemein verwendet oder auch Haken ausgebildet werden kann, weist einen Gewindeschaft 1 und einen Kopf 2 auf. Der Kopf 2 weist eine sich in Richtung der Längsachse 3 der Schraube erstreckende U-förmige Ausnehmung 4 auf, die eine vorbestimmte Tiefe aufweist. An den dadurch gebildeten beiden Schenkeln 5, 6 ist vom freien Ende 7 der Schenkel her ein Innengewinde 8 vorgesehen. Passend zu dem Innengewinde 8 ist eine Schraube 9 vorgesehen, deren Außengewinde so ausgebildet ist, daß es mit dem Innengewinde 8 zusammenwirkt.

Im Betrieb wird ein mit der Schraube zu verbindender Stab 10 in die U-förmige Ausnehmung 4 so eingelegt, daß der Stab auf dem Grund der U-förmigen Ausnehmung zu liegen kommt. Dann wird die Schraube 9 so weit eingeschraubt, daß sie einen ausreichenden Arretierungsdruck auf den in der U-förmigen Ausnehmung liegenden Stab 10 ausübt.

Wie deutlicher aus den Fig. 2 und 3 ersichtlich ist, sind das Innengewinde in dem Kopf 2 und das Außengewinde der Schraube 9 nach Art eines Sägengewindes ausgebildet. Die Schraube 9 weist auf der dem zu fixierenden Stab 10 abgewandten Oberfläche 11 einen Schlitz oder eine hexagonale Ausnehmung zum Eingreifen eines Schraubendrehers auf.

In dem Gewinde bilden jeweils zwei benachbarte Teilflanken 12, 13 einen Gewindegang. Die beiden Teilflanken 12, 13 schließen den Flankenwinkel β ein. Die dem freien Ende 7 zugewandte Flankenseite 13 schließt mit einer sich senkrecht zur Längsachse 3 erstreckenden Ebene 40, die die Flankenseite 13 an dem Gewindegrund 23 schneidet, einen positiven Winkel β₁ ein (siehe Fig. 3). Die dem freien Ende abgewandte Teilflanke 12 ist so geneigt, daß sie in der in Fig. 3 gezeigten Weise mit einer entsprechenden Ebene 40 einen negativen Winkel β₂ einschließt. Wie aus den Figuren ersichtlich ist, ist das Gewinde der Schraube 9 entsprechend ausgeführt, so daß die Schraubengänge der Schraube 9 passend in das Innengewinde 8 eingreifen. Bei der Schraube 9 bildet also jede dem freien Ende 7 bzw. der Oberfläche 11 zugewandte Flanke 26 den negativen Winkel β₂, der mit der entsprechenden Flanke 12 zusammenwirkt.

Die in Fig. 4 gezeigte Ausführungsform stimmt in der Ausbildung des Innengewindes 8 und der damit zusammenwirkenden Schraube 9 und des Stabes 10 identisch überein. Übereinstimmende Merkmale sind mit den gleichen Bezugszeichen gekennzeichnet.

Anders als bei der ersten Ausführungsform handelt es sich hier um eine sogenannte Polyaxialschraube, bei der der Gewindeschaft 1 über einen sphärischen Kopf 14 mit einem Aufnahmeteil 15 verbunden ist. Dieses weist an seinem einen Ende eine axialsymmetrisch ausgerichtete erste Bohrung 16, deren Durchmesser größer als der des Gewindeabschnittes 1 und kleiner als der des Kopfes 14 ist. Das Aufnahmeteil weist ferner eine koaxiale zweite Bohrung 17 auf, die auf dem der ersten Bohrung gegenüberliegenden Ende offen ist und deren Durchmesser so groß ist, daß das Schraubenelement durch das offene Ende mit seinem Gewindeabschnitt durch die erste Bohrung hindurch und mit dem Kopf 14 bis zum Grund der zweiten Bohrung führbar ist. Zwischen der ersten und der zweiten Bohrung ist ein kleiner koaxialer Abschnitt vorgesehen, der unmittelbar an die erste Bohrung angrenzt und zum offenen Ende hin sphärisch ausgebildet ist, wobei der Radius im wesentlichen gleich dem Radius des kugelsegmentförmigen Abschnittes des Kopfes 14 ist. Angrenzend an die freien Enden 7 der Schenkel 5, 6 ist wiederum das Innengewinde 8 vorgesehen. Ferner weist das Aufnahmeteil eine zur Mitte des Teiles symmetrisch angeordnete U-förmige Ausnehmung 18 auf, deren Grund zu der ersten Bohrung hin gerichtet ist, deren beiden Seitenschenkel 5, 6 sich zu dem offenen Ende 7 hin erstrecken. Ferner ist in der zweiten Bohrung 17 ein Druckelement eingesetzt, welches auf dem sphärischen Kopf 14 aufsitzt. Im Betrieb wird der Stab 10 in die U-förmige Ausnehmung eingesetzt. Anschließend wird die Schraube 9 in der gleichen Weise wie in Fig. 2 gezeigt, eingedreht und soweit geschraubt, bis sie einen gewünschten Druck auf das Druckelement 19 und damit auf den Kopf 14 zum Arretieren desselben ausübt.

Anstelle der oben beschriebenen Ausführungen, bei denen der Kopf bzw. das Aufnahmeteil jeweils mit einer Knochenschraube verbunden sind, können der Kopf bzw. das Aufnahmeteil auch mit einem Haken verbunden sein, wie er in der Wirbelsäulenchirurgie zum Einhaken hinter Knochenvorsprüngen der Wirbelsäule verwendet wird.

Wenn oben von einem Sägengewinde die Rede ist, so soll damit lediglich die Richtung der Teilflanken gekennzeichnet werden. Eingeschlossen sein sollen sowohl Gewinde mit abgerundeten Spitzen und abgerundetem Grund, Spitzengewinden und Spitzengewindegrund und flachen Gewinderändern und flachem Gewindegrund, wie dies auch bei Spitzgewinde, Rundgewinde und Trapezgewinde der Fall ist.

Während bei gekannten Schrauben mit einem metrischen Gewinde, in dem die Schraube in der Fig. 2 gezeigten Weise auf einen Stab drückt, eine Kraftkomponente zur offenen Seite 11 der Schraube und eine zweite Kraftkomponente jeweils nach außen wirkt, wirkt bei dem oben beschriebenen erfindungsgemäßen Gewinde wiederum eine Komponente zum freien Ende 7 hin, die zweite Komponente jedoch in Richtung der Längsachse 3. Das hat zur Folge, daß die freien Schenkel 5, 6 nicht nach außen gedrückt, sondern zur Symmetrieachse hin gezogen werden.

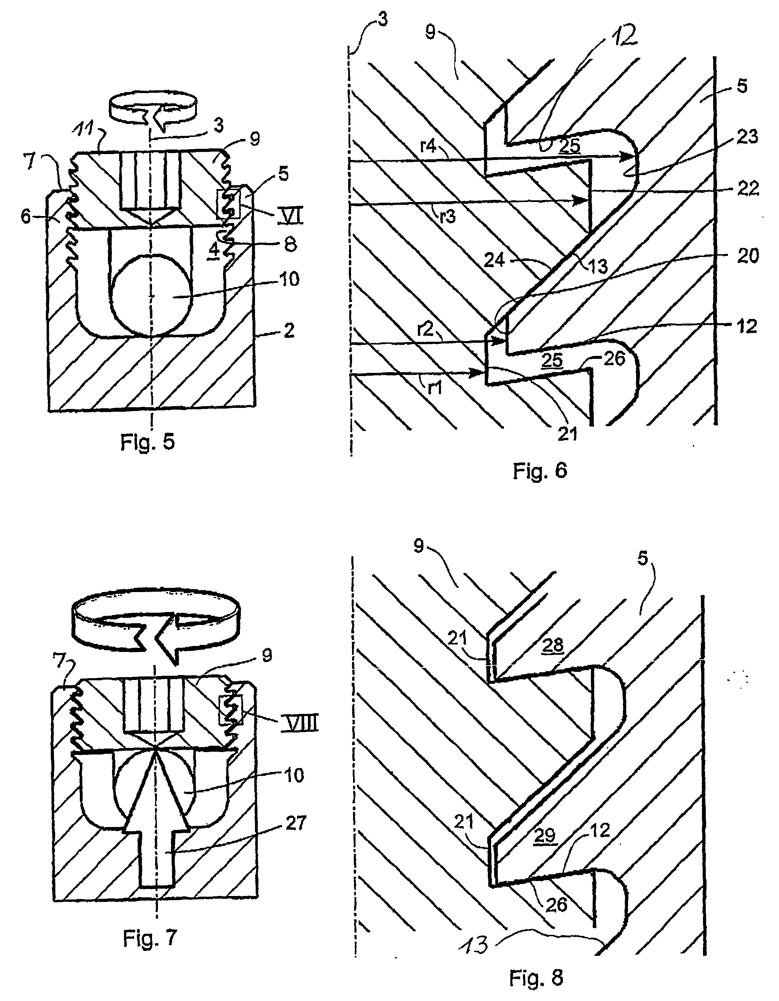

In Fig. 5 ist wie in Fig. 2 der Kopf der in Fig. 1 gezeigten Pedikelschraube mit eingesetztem Stab und nur anfangs eingesetzter Innenschraube 9 gezeigt. In Fig. 6 ist der Ausschnitt VI aus Fig. 5 im Detail größer dargestellt. Wie sich aus dieser vergrößerten Darstellung ergibt, sind die Innenschraube 9 und das Außengewinde der Schenkel 5 wie folgt relativ zueinander bemessen: Der Radius r₁ von der mit der Längsachse 3 zusammenfallenden Schraubenmitte bis zum Gewindegrund 21 der Schraube 9 ist kleiner als der Radius r₂ von der mit der Symmetrieachse 3 zusammenfallenden Mittenachse des Halteelementes bis zu seiner Wendelspitze 20. Der Radius r₃ von der mit der Längsachse 3 zusammenfallenden Schraubenmitte bis zur Wendelspitze 22 der Schraube 9 ist kleiner als der Radius r₄ von dem mit der Längsachse 3 zusammenfallenden Mittenachse des Halteelementes bis zum Gewindegrund 23 seines Innengewindes.

Wie insbesondere aus Fig. 6 ersichtlich ist, gilt beim unbelasteten Ineingriffbringen von Schraube 9 und freien Schenkeln 5, 6 des Halteelementes beim Aneinanderliegen der zusammenwirkenden Flanken 13 des Innengewindes und 24 der Schraube mit positiven Flankenwinkeln ein Spalt 25 zwischen den jeweils einander zugewandten Flanken 12, 26 mit negativem Flankenwinkel β₂.

Die Fig. 7 zeigt den gleichen Gegenstand wie in den Figuren 5 und 6, wobei die Schraube 9 jetzt so weit nach innen geschraubt ist, daß sie auf den Stab 10 drückt und dadurch eine durch den Pfeil 27 angedeutete Gegenkraft auf die Schraube 9 wirkt. Als Ergebnis davon gelangen, wie aus Fig. 8 ersichtlich ist, dadurch die jeweils einander zugewandten Flanken 12, 26 mit negativem Flankenwinkel β₂ aufeinander, wodurch die Wendel 28, 29 des Innengewindes in der in Fig. 8 gezeigten Weise zu dem jeweiligen Grund 21 der Gewindegänge des Gewindes der Schraube 9 hin nach innen gezogen werden mit der Folge, daß die freien Schenkel nicht nach außen, sondern nach innen vorgespannt werden.

In einer bevorzugten Ausführungsform beträgt der Unterschied zwischen dem Maß der Radien r₁ und r₂ etwa 1 bis 5 % des Gewindeaußendurchmessers bzw. 0,1 bis 0,5 mm bei einem Schraubendurchmesser von 5 bis 12 mm.

## Patentansprüche

1. Element mit einem Schaft (1) und einem damit verbundenen Halteelement (2) zum Verbinden mit einem Stab (10) zur Verwendung in der Wirbelsäulen- bzw. Unfallchirurgie, wobei das Halteelement (2) eine einen U-förmigen Querschnitt aufweisende Ausnehmung (4) zur Aufnahme des Stabes (10) mit zwei an einem Ende (7) freien Schenkeln (5,6) und einem Innengewinde (8) an den freien Schenkeln (5,6) und eine mit dem Innengewinde (8) zusammenwirkende Schraube (9), die auf den Stab (10) einwirkt, aufweist, wobei bei dem Innengewinde (8) mit zwei aneinandergrenzenden Teilflanken (12, 13) die dem freien Ende (7) abgewandten Teilflanken (12) mit einer jeweils durch den Gewindegrund (23) gehenden und sich senkrecht zur Schraubenachse (3) erstreckenden Ebene einen negativen Winkel β₂ einschließen, und wobei die mit dem Innengewinde (8) zusammenwirkende Schraube (9) ein dazu passendes Außengewinde aufweist, der Radius (r₁) von der Schraubenmitte (3) bis zum Gewindegrund (21) der Schraube (9) kleiner ist als der Radius (r₂) von der Mittenachse (3) des Halteelementes bis zu seiner Wendelspitze (20), und der Radius (r₃) von der Schraubenmitte (3) bis zur Wendelspitze (22) der Schraube (9) kleiner ist als der Radius (r₄) von der Mittenachse (3) des Halteelementes bis zum Gewindegrund (23) seines Innengewindes **dadurch gekennzeichnet daß** beim unbelasteten Ineingriffbringen von Schraube (9) und freien Schenkeln (5, 6) des Halteelementes beim Aneinanderliegen der zusammenwirkenden Flanken (13, 24) mit positivem Flankenwinkel β₁ ein Spalt (25) zwischen den jeweils einander zugewandten Flanken (12, 26) mit negativem Flankenwinkel β₂ vorgesehen ist.

2. Element nach Anspruch 1, **dadurch gekennzeichnet, daß** der Grund (23) des Gewindes der freien Schenkel (5, 6) jeweils abgerundet ausgebildet ist.

## Claims

1. Element with a shank (1) and with a retention element (2) which is connected to the latter and which is intended for connection to a rod (10), the retention element (2) comprising a recess (4) of U-shaped cross section, which receives the rod (10), with two arms (5, 6) free at one end (7), and with an internal thread (8) on the free arms (5, 6), and a screw (9) which cooperates with the internal thread (8) and acts on the rod (10), where, in the internal thread (8) with two adjoining flank parts (12, 13), the flank parts (12) facing away from the free end (7) enclose a negative angle β₂ with a plane passing through the thread root (23) and extending perpendicular to the screw axis (3), and where the screw (9) cooperating with the internal thread (8) has an external thread matching the latter, the radius (r1) from the screw centre (3) to the thread root (21) of the screw (9) is smaller than the radius (r2) from the centre axis (3) of the retention element to its helix tip (20), and the radius (r3) from the screw centre (3) to the helix tip (22) of the screw (9) is smaller than the radius (r4) from the centre axis (3) of the retention element to the thread root (23) of its internal thread, **characterized in that** upon unloaded engagement of the screw (9) in the free arms (5, 6) of the retention element, and with the cooperating flanks (13, 24) lying against one another with a positive flank angle β₁, a gap (25) is provided between the mutually facing flanks (12, 26) with negative flank angle β₂.

2. Element according to Claim 1, **characterized in that** the root (23) of the thread of the free arms (5, 6) in each case has a rounded shape.

## Revendications

1. Elément avec une tige (1) et un élément de retenue (2) connecté avec la tige pour la liaison avec une barre (10) pour une utilisation dans la chirurgie de la colonne vertébrale ou la chirurgie des accidents, l'élément de retenue (2) présentant un évidement (4) présentant une section en forme de U pour le logement de la barre (10) avec deux branches (5, 6) libres sur une extrémité (7) et un filet interne (8) sur les branches (5, 6) libres et une vis (9) coopérant avec le filet interne (8), qui agit sur la barre (10), moyennant quoi, sur le filet interne (8) avec deux flancs partiels (12, 13) contigus, les flancs partiels (12) opposés à l'extrémité libre (7) forment un angle β₂ négatif avec un plan passant respectivement par le fond du filet (23) et s'étendant perpendiculairement à l'axe de vis (3), et moyennant quoi la vis (9) coopérant avec le filet interne (8) présente un filet externe adapté à la vis, le rayon (r₁) allant du centre de la vis (3) jusqu'au fond de filet (21) de la vis (9) étant inférieur au rayon (r₂) allant de l'axe central (3) de l'élément de retenue jusqu'à son extrémité hélicoïdale (20), et le rayon (r₃) allant du centre de la vis (3) jusqu'à l'extrémité hélicoïdale (22) de la vis (9) étant inférieur au rayon (r₄) allant de l'axe central (3) de l'élément de retenue jusqu'au fond du filet (23) de son filet interne, **caractérisé en ce que**, avec l'engrènement non chargé de la vis (9) et des branches (5, 6) libres de l'élément de retenue, il est prévu lors du déplacement contigu des flancs (13, 24) coopérants avec un angle de flanc β₁ positif, une fente (25) entre les flancs (12, 26) respectivement tournés les uns vers les autres avec un angle de flanc β₃ négatif.

2. Elément selon la revendication 1, **caractérisé en ce que** le fond (23) du filet des branches (5, 6) libres est conçu à chaque fois de façon arrondie.
